# EUROPEAN PATENT APPLICATION

(11) **EP 4 667 903 A1**
(43) Date of publication of application: **24.12.2025**
(21) Application number: 23922318.3
(22) Date of filing: 07.10.2023
(51) Int. Cl.: G01N 15/14, G01N 21/64

(54) **HIGH-THROUGHPUT PARALLEL TESTING METHOD BASED ON IMAGING FLOW CYTOMETRY AND SPATIALLY CODED REAGENT**

(30) Priority: 14.02.2023 CN 202310113396
(71) Applicant: Fairy Life Sciences (Wuhan) Co., Ltd., Wuhan, Hubei 430075 (CN)
(72) Inventor: ZHAO, Jingjing, Zhengzhou, Henan 450046 (CN); DU, Lin, Zhengzhou, Henan 450046 (CN)
(74) Representative: Metida
(86) International application number: PCT/CN2023/123197
(87) International publication number: WO 2024/169200

(57) **Abstract**

A high-throughput parallel testing method based on imaging flow cytometry and spatially coded reagents combines the high-throughput characteristic of an imaging flow cytometer and the high coding capacity characteristic of spatially coded reagents, thereby achieving high-throughput parallel testing of nucleic acid sequencing, protein testing and single cell analysis, and being capable of being used for liquid biopsy or other biomedical use requirements for performing high-throughput synchronous testing on different biomarkers.

## Description

### CROSS-REFERENCE TO RELATED APPLICATIONS

The present application claims priority to Chinese Patent Application No. 202310113396.X, filed on February 14, 2023, the entire contents of which are incorporated herein by reference.

### TECHNICAL FIELD

The present invention relates to the fields of instrument analysis, in particular to a method for high-throughput parallel detection based on imaging flow cytometry and spatially coded reagents.

### BACKGROUND

Biomedical detection of liquid biopsy requires high-throughput parallel detection of different biological particles or biomarkers in a suspended state to improve the speed and accuracy of detection. Common analytes include nucleic acids, proteins, and single cells, and common detection means include flow cytometry and fluorescence microscopy. Flow cytometry can realize the high-throughput detection of biological particles at a throughput up to a range of 10³-10⁵ particles per second, and detect scattered light and fluorescence signals of biological particles. However, this method does not have two-dimensional spatial resolution or imaging capabilities. Furthermore, the number of assays carried out in a flow cytometer is limited by the spectral resolution capabilities. Even with the most advanced spectral flow cytometers or fluorescent intensity-encoded microspheres, the number for parallel detection capacity still cannot exceed 50. Fluorescence microscopy can image biological particles, typically sequencing free nucleic acid fragments by the next-generation sequencing (NGS) technology, analyzing free proteins by enzyme-linked immunosorbent assay (ELISA), analyzing nucleic acids within single cells by fluorescence in situ hybridization (FISH), and analyzing proteins within single cells by fluorescent labeling. Fluorescence microscopy combined with spatially coded reagents can enable the parallel detection of 10²-10³ different objects to be detected. However, fluorescence microscopy requires fixing objects to be detected on a substrate and the number of objects to be detected within a single imaging field is limited, so the operation is complex and the detection throughput is far lower than that of flow cytometry. In summary, if a method for the detection of biological particles can achieve parallel detection of 10²-10⁵ different objects to be detected at a throughput of 10²-10⁵ analytes per second and is also applicable to the detection of nucleic acids, proteins, and single cells, this detection method can greatly improve the efficiency of detection and the accuracy of analysis results.

Imaging flow cytometer relates to newly developed techniques for analysis of biological particles in recent years. By combining flow technology and high-speed microscopy technology, it realizes multi-channel fluorescence and scattered light imaging of biological particles in a high-speed flow state, and has the high-throughput characteristics of conventional flow cytometers and the two-dimensional spatial resolution capability of microscopic imaging. However, the number of parallel assays available with existing imaging flow cytometers is still limited by the spectral width of each fluorescent reagent. Their parallel detection capabilities, similar to those of conventional flow cytometers, still cannot exceed the parallel detection of 50 different analytes. In the next-generation sequencing technology, fluorescent spatially coded reagents are widely used. By spatially coding fluorophores, they can generate 10²-10³ different codes, and can identify different spatial codes using high-resolution microscopic imaging. Each code can be uniquely assigned to an object to be detected, and thus enables extremely high parallel detection capabilities. However, the spatially coded microspheres used in the next-generation sequencing technology are extremely small in size, typically each in a size on the order of 10-100 nm. Therefore, they cannot be effectively resolved by imaging flow cytometry and have not been used for imaging flow cytometry-based detection. Given the above analysis, there is an urgent need to design spatially coded reagents that can be effectively resolved by imaging flow cytometers, which would combine the high throughput of imaging flow cytometers with the high coding capacity of spatially coded reagents, thereby enabling high-throughput parallel detection of multiple biological particles.

### SUMMARY OF THE INVENTION

The present invention is to at least solve one of the technical problems in the relevant art to some extent. Accordingly, one purpose of the present invention is to provide a method for high-throughput parallel detection based on imaging flow cytometry and spatially coded reagents. The method combines the high-throughput characteristic of imaging flow cytometers with the high coding capacity characteristic of spatially coded reagents. It can achieve the high-throughput parallel detection of nucleic acid sequencing, protein detection and single-cell analysis, and is useful for liquid biopsy or other biomedical applications that require high-throughput synchronous detection of different biomarkers.

As such, a first aspect of the present invention provides a method for high-throughput parallel detection of biological particles, comprising:
(1) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library;
(2) constructing a biological particle library using known biological particles, and assigning one unique spatially coded reagent in the coding sequence library to each biological particle in the biological particle library to establish one-to-one correspondences between the coding sequence library and the biological particle library;
(3) labeling biological particles to be detected with the spatially coded reagents to form objects to be detected; and
(4) placing the objects to be detected in a suspended state in flow channels of an imaging flow cytometer for detection to obtain information of the biological particles to be detected.

The method for high-throughput parallel detection of biological particles provided by the present invention is based on imaging flow cytometry and spatially coded reagents. The spatially coded reagents can be composed of spatially arranged fluorescent microspheres of different colors. Depending on the colors and spatial positions of the fluorescent microspheres, the spatially coded reagents can generate different coding sequences, thereby forming a coding sequence library. For example, if there are fluorescent microspheres of four different colors and a spatially coded reagent 2 consists of four microspheres, 4⁴ or 256 different coding sequences can be formed; if there are fluorescent microspheres of five different colors and a spatially coded reagent consists of four microspheres, 5⁴ or 625 different coding sequences can be formed; if there are fluorescent microspheres of five different colors and a spatially coded reagent consists of five microspheres, 5⁵ or 3125 different coding sequences can be formed; if there are fluorescent microspheres of ten different colors and a spatially coded reagent consists of four or five microspheres, 10⁴ 10⁵ different coding sequences can be formed. Compared with spatial coding microspheres in an extremely small size, the spatially coded reagents provided by the present invention have a high coding capacity and can be used in imaging flow cytometers to achieve high-throughput parallel detection of multiple biological particles.

According to the embodiments of the present invention, the biological particles in step (2) comprise at least one of nucleic acids, proteins, bacteria, viruses, exosomes and single cells.

According to the embodiments of the present invention, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

According to the embodiments of the present invention, in step (3), the color type and spatial position arrangement of the microspheres labeled with the spatially coded reagents are consistent with the color type and spatial position arrangement of the microspheres of the spatially coded reagent in the coding sequence library corresponding to the biological particles to be detected.

According to the embodiments of the present invention, the liquid flows in the flow channels of the imaging flow cytometer in step (4) have a central flow velocity of 0.1-20 m/s.

According to the embodiments of the present invention, in step (4), the information of the biological particles to be detected is acquired by obtaining the color type and spatial position of the microspheres of the spatially coded reagent associated with the biological particles to be detected, and using the correspondences between the coding sequence library and the biological particle library.

A second aspect of the present invention provides a method for high-throughput sequencing, comprising:
selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein one end of the spatially coded reagent is connected to a nucleic acid single strand a, and the end of the nucleic acid single strand a that is not connected to the spatially coded reagent comprises complementary bases paired with a portion of bases in a single-stranded nucleic acid to be detected;
connecting a fluorophore with a nucleic acid single strand b to prepare a reporter probe, wherein one end of the nucleic acid single strand b that is not connected to the fluorophore comprises complementary bases paired with a portion of the bases in the single-stranded nucleic acid to be detected, and in the single-stranded nucleic acid to be detected, the portion complementary to the nucleic acid single strand a does not overlap with the portion complementary to the nucleic acid single strand b;
constructing single-stranded nucleic acid fragments to be detected into a nucleic acid sequence library, and assigning one unique spatially coded reagent in the coding sequence library to each nucleic acid fragment in the nucleic acid sequence library;
simultaneously combining the spatially coded reagents and the reporter probe with single-stranded nucleic acid sequences to be detected to form objects to be detected, wherein the spatially coded reagents are types of spatially coded reagents corresponding to the types of all nucleic acid sequences in the nucleic acid sequence library; and
placing the objects to be detected in a suspended state in flow channels of an imaging flow cytometer for detection to obtain information of nucleic acid sequences using the correspondences between the coding sequence library and the nucleic acid sequence library when the color type and spatial position of the microspheres labeled with the spatially coded reagents and the fluorophore are detected simultaneously.

According to embodiments of the present invention, the liquid flows in the flow channels of the imaging flow cytometer in step (5) have a central flow velocity of 0.1-20 m/s.

According to the embodiments of the present invention, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

A third aspect of the present invention provides a method for high-throughput detection of proteins, comprising:
Method 1: (1) connecting an antibody and a nucleic acid single strand c to prepare a capture probe, wherein the antibody specifically binds to a target protein;
   (2) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein one end of the spatially coded reagent is connected to a nucleic acid single strand d, and the end of the nucleic acid single strand d that is not connected to the spatially coded reagent comprises complementary bases paired with a portion of the bases in the nucleic acid single strand c;
   (3) connecting a fluorophore with a nucleic acid single strand e to prepare a reporter probe, wherein the end of the nucleic acid single strand e that is not connected to the fluorophore comprises complementary bases paired with a portion of the bases in the nucleic acid single strand c;
      and in the nucleic acid single strand c, the portion complementary to the nucleic acid single strand d does not overlap with the portion complementary to the nucleic acid single strand e;
   (4) constructing proteins to be detected into a protein library, and assigning one unique spatially coded reagent in the coding sequence library to each protein in the protein library;
      simultaneously combining the spatially coded reagents and the reporter probe with the nucleic acid single strand c in the capture probe to form objects to be detected; and
      placing the objects to be detected in a suspended state in flow channels of an imaging flow cytometer for detection to obtain information of proteins using the correspondences between the coding sequence library and the protein library when the color type and spatial position of the microspheres labeled with the spatially coded reagent and the fluorophore are detected simultaneously;
or Method 2: (1) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein the spatially coded reagents are connected to an antibody for specific binding to a target protein;
   (2) constructing proteins to be detected into a protein library, and assigning one unique spatially coded reagent in the coding sequence library to each protein in the protein library;
   (3) connecting the spatially coded reagents to the target protein on the cell surface through an antibody to form objects to be detected; and
   (4) placing the objects to be detected in a suspended state in flow channels of an imaging flow cytometer for detection to obtain information of proteins using the correspondences between the coding sequence library and the protein library when the color type and spatial position of the microspheres labeled with the spatially coded reagents are detected.

According to the embodiments of the present invention, the liquid flows in the flow channels of the imaging flow cytometer have a central flow velocity of 0.1-20 m/s.

According to the embodiments of the present invention, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

A fourth aspect of the present invention provides a method for detecting single cells, comprising:
specifically binding the surface of single cells to be detected to an anchor protein;
selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein the spatially coded reagents are connected to an antibody for specific binding to the anchor protein;
constructing cells to be detected into a cell library, and assigning one unique spatially coded reagent in the coding sequence library to each cell in the cell library;
connecting the spatially coded reagents to the anchor protein on the cell surface through an antibody to form objects to be detected; and
placing the objects to be detected in a suspended state in flow channels of an imaging flow cytometer for detection to obtain information of single cells using the correspondence between the coding sequence library and the cell library when the color type and spatial position of the microspheres labeled with the spatially coded reagents are detected.

According to the embodiments of the present invention, the liquid flows in the flow channels of the imaging flow cytometer in step (5) have a central flow velocity of 0.1-20 m/s.

According to the embodiments of the present invention, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

A fifth aspect of the present invention provides a method for high-throughput parallel detection of a liquid biopsy sample, comprising: detecting biological particles contained in the liquid biopsy sample using the method of the first aspect.

According to the embodiments of the present invention, the liquid biopsy sample comprises at least one of cfDNA, a cell, free RNA, DNA, an exosome, and a free protein.

According to the embodiments of the present invention, the method comprises sequencing nucleic acids contained in the liquid biopsy sample using the method for high-throughput sequencing of the second aspect;
detecting proteins contained in the liquid biopsy sample using the method for high-throughput detection of proteins of the third aspect;
detecting cells contained in the liquid biopsy sample using the method for single-cell detection of the fourth aspect;
wherein the spatial encoding reagents in the method for high-throughput sequencing, the method for high-throughput detection of proteins and the method for single-cell detection have different colors and/or spatial positions of microspheres.

A sixth aspect of the present invention provides a method of preparing the spatially coded reagents according to any one of the methods of the first, second, third, fourth, and fifth aspects, the method comprising:
selecting fluorescent labels having small fluorescence spectral overlap, and labeling the surface of microspheres with the fluorescent labels, wherein each microsphere comprises only one fluorescent label on the surface;
linking the surface of the labeled microspheres obtained in step (1) with DNA single-strands; and
connecting the labeled microspheres by DNA complementary base pairing to obtain the spatially coded reagents.

According to the embodiments of the present invention, the fluorescent labels in step (1) comprise at least one selected from fluorescein, quantum dots, fluorescent proteins and polymer dyes;

According to the embodiments of the present invention, the fluorescent labels in step (1) are selected from 3 to 20 types.

According to the embodiments of the present invention, the microspheres in step (1) comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

According to the embodiments of the present invention, the spatially coded reagents are further modified, and the modification comprises connecting the spatially coded reagents to a nucleic acid single-stranded DNA or connecting the spatially coded reagents to an antibody.

A seventh aspect of the present invention provides use of imaging flow cytometry and spatially coded reagents in the detection of biological particles, wherein the method for the detection biological particles comprises the method of high-throughput parallel detection of biological particles provided in the first aspect.

According to the embodiments of the present invention, the biological particles comprise at least one of nucleic acids, proteins, bacteria, viruses, exosomes and single cells.

Additional aspects and advantages of the present invention will be set forth in part in the description which follows, and in part will be evident from the description which follows, or will be learned by practice of the present invention.

### BRIEF DESCRIPTION OF THE DRAWINGS

The above and/or additional aspects and advantages of the present invention will become apparent and easily understood with reference to the following description of the embodiments made in the drawings, in which:
FIG. 1 illustrates the methods of preparing spatially coded reagents and constructing a coding sequence library in Example 1 of the present invention;
FIG. 2 illustrates the method for high-throughput parallel detection of biological particles in Example 2 of the present invention;
FIG. 3 illustrates the detection of biological particles in a water body sample based on the method for high-throughput parallel detection of biological particles in Example 2 of the present invention;
FIG. 4 illustrates the method for high-throughput sequencing in Example 3 of the present invention;
FIG. 5 illustrates the process for gene fragment sequencing based on the method for high-throughput sequencing in Example 3 of the present invention;
FIG. 6 illustrates the method for high-throughput protein detection of free proteins in Example 4 of the present invention;
FIG. 7 illustrates the method for high-throughput protein detection of cell surface proteins in Example 4 of the present invention;
FIG. 8 illustrates the simplified method of high-throughput protein detection for cell surface proteins in Example 4 of the present invention;
FIG. 9 illustrates the method for single-cell detection in Example 5 of the present invention;
FIG. 10 illustrates the analysis of the toxicity of candidate drugs based in the method for single-cell detection in Example 5 of the present invention;
FIG. 11 illustrates the method for high-throughput parallel detection of a liquid biopsy sample in Example 6 of the present invention.

### DETAILED DESCRIPTION

Hereinafter, embodiments of the present invention will be described in detail. The embodiments described below are exemplary and are only used to explain the present invention, and shall not be construed as limitations to the present invention.

It should be noted that the terms "first" and "second" are used merely for the purpose of description, and shall not be construed as indicating or implying relative importance or implicitly indicating a quantity of indicated technical features. Thus, the features defined with "first" and "second" may explicitly or implicitly include one or more of the features. Further, in the description of the present invention, "more" indicates two or above unless otherwise specified.

The endpoints and any values of the ranges disclosed herein are not limited to the exact ranges or values, and these ranges or values should be understood to include values close to these ranges or values. As for numerical ranges, the endpoint values of the various ranges, the endpoint values and the individual point value of the various ranges, and the individual point values may be combined with one another to produce one or more new numerical ranges, which should be regarded as having been specifically disclosed herein.

In order to make the present invention more easily understood, certain technical and scientific terms are specifically defined below. Unless specifically defined elsewhere in this document, all other technical and scientific terms used herein have the meaning as commonly understood by one of ordinary skill in the art to which the present invention belongs.

Herein the terms "include" or "comprise" are open-ended expressions, that is, including the contents specified in the present invention but not excluding other contents.

### Definitions and Explanations of Terms

Unless otherwise indicated, the definitions of groups and terms in the specification and claims of the present application, including definitions provided as examples, exemplary definitions, preferred definitions, definitions disclosed in tables, and definitions of specific compounds in the examples, may be arbitrarily combined and incorporated with one another. The group definitions and compound structures resulting from such combinations and incorporations shall be within the scope of disclosure contained in the specification of the present application.

Herein, the term "antibody" is used in the broadest sense and includes fully assembled antibodies, tetrameric antibodies, monoclonal antibodies, polyclonal antibodies, multispecific antibodies (e.g., bispecific antibodies), and antigen-binding antibody fragments (e.g., Fab', F'(ab)2, Fv, single-chain antibodies, diabodies, Fab), as long as they exhibit the desired biological activities.

The solutions of the present disclosure will be explained in conjunction with the examples below. Those skilled in the art will understand that the following examples are only used to illustrate the present disclosure and should not be regarded as limiting the scope of the present disclosure. If no specific techniques or conditions are specified in the examples, the techniques or conditions described in literatures of the art or the product instructions shall be followed. The reagents and instruments in use for which the manufacturers are not specified are all conventional products that can be purchased commercially.

### EXAMPLE 1. PREPARATION OF SPATIALLY CODED REAGENTS AND CONSTRUCTION OF CODING SEQUENCE LIBRARY

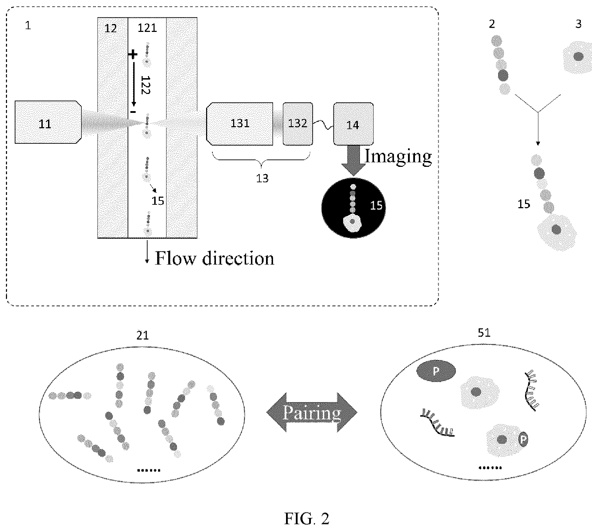

Drugs that block the proliferation of cancer cells are studied. The effects of various candidate drugs on the proliferation of cancer cells and healthy tissue cells are observed. Healthy tissue cells and cancer cells are taken in the same number and cultured in a suitable environment after various candidate drugs are added, and control groups with no addition of any drugs are set up. After a period of culture, such as 5 to 9 days, the cultured cells are prepared into a single-cell suspension, and the cell nuclei are stained with SYTO16. Subsequently, each single cell sample is imaged through an imaging flow system to collect bright-field and nucleus fluorescence images of the cells. As shown in FIG. 2, according to the morphology of cell nuclei and the cell contour profiles in the bright field, an AI algorithm can distinguish the G0/G1, S, G2, and M stages in the cell cycle. The healthy tissue cells with no addition of any drugs are taken as the control group A, and the number and relative proportion of cells in different cell cycle stages are counted. The cancer cells with no addition of any drugs are taken as the control group B, and the number and relative proportion of cells in different cell cycle stages are counted. Since cancer cells proliferate faster than healthy cells, cancer cells after culture have a greater absolute number than healthy tissue cells, and cells in the division phase are in a higher proportion. The images and statistical data of candidate drug C on healthy tissue cells and cancer cells are observed. If the images and statistical data of candidate drug C on healthy tissue cells are close to those of the control group A, it is indicated that candidate drug C is not toxic to healthy cells; if abnormal, such as a decrease in the absolute number of cells and/or a decrease in the proportion of cells in the division phase, it is indicated that candidate drug C has toxic side effects on healthy tissue cells. It is necessary to further study the effects of candidate drug C on healthy cells or exclude it from the candidate list. If the images and statistical data of candidate drug C on cancer cells are close to those of the control group B, it is indicated that candidate drug C cannot effectively block the proliferation of cancer cells and is not a desired candidate drug; if the absolute number of cancer cells upon the action of candidate drug C decreases significantly, and a number of cancer cells are blocked in one of the G0/G1, S, G2, and M stages, it is indicated that candidate drug C can effectively block the proliferation of cancer cells, and the drug target can be further analyzed according to the blocking position. Finally, several drugs that can effectively block the proliferation of cancer cells and have no obvious side effects on healthy tissue cells are selected, and these drugs are studied more deeply and extensively to finally determine the optimal drug. Generally, for small-molecule compound drugs, the initial candidate drugs can be as many as thousands to hundreds of thousands, so high-throughput imaging flow analysis plays an irreplaceable role in improving the efficiency of drug screening and accelerating drug development.

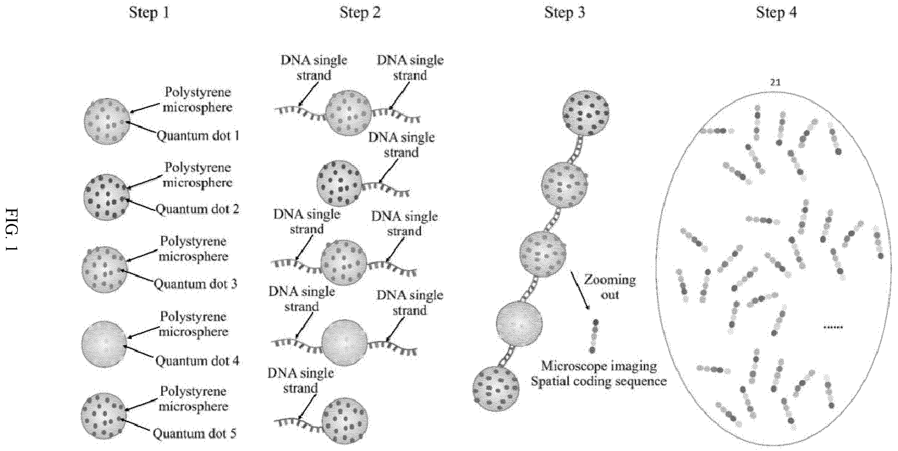

The specific steps are shown in FIG. 1. In the first step, five types (or more, e.g., 3-20) quantum dots with minimal fluorescence spectral overlap are selected and labeled the surface of polystyrene microspheres via a polymerization reaction. Each polystyrene microsphere contains 10²-10⁴ quantum dots on the surface, and each polystyrene microsphere contains only one type of quantum dots so that each polystyrene microsphere has only one color; In the second step, one or two different DNA single strands are attached to the surface of the polystyrene microspheres; In the third step, the polystyrene microspheres of different colors are connected in a specific spatial order through pairing of DNA complementary strands; In the fourth step, spatially coded reagents are arranged according to the color and spatial position of the microspheres to form a coding sequence library 21.

The size of polystyrene microspheres is selected based on the spatial resolution of an imaging flow cytometer. For an imaging flow cytometer having a resolution of 1 µm, polystyrene microspheres in a size of 1 µm can be selected, and two adjacent polystyrene microspheres have a spacing of 0.5 µm, thereby meeting the requirement for accurate identification of the spatially coded reagents. The prepared spatially coded reagents are arranged according to different colors and spatial positions of microspheres, forming a coding sequence library, which can be used for nucleic acid sequence detection, protein detection and specific identification of single cells.

### EXAMPLE 2. SPATIALLY CODED REAGENTS FOR HIGH-THROUGHPUT PARALLEL DETECTION OF BIOLOGICAL PARTICLES

The detection method is based on imaging flow cytometry and spatially coded reagents in Example 1. The specific steps are shown in FIG. 2.

Specifically, the detection method primarily comprises an imaging flow cytometer 1, spatially coded reagents 2, and biological particles 3 to be detected. The imaging flow cytometer mainly comprises a laser illumination unit 11, a flow chamber 12, an imaging detection unit 13, and a signal processing unit 14. The laser illumination unit 11 is responsible for shaping one or more laser beams into the required illumination spot. The flow chamber 12 is responsible for focusing suspended objects 15 to be detected at the center of flow channels 121 to ensure that the suspended objects 15 to be detected can be illuminated by the illumination spot and transmit scattered light and fluorescence. The liquid flow in the flow channels 121 of the flow chamber has a central flow velocity in a range of 0.1-20 m/s, at a throughput of 10²-10⁵ analytes per second. An optical system 131 in the imaging detection unit 13 is responsible for collecting the scattered light and fluorescence of each particle, and a photoelectric conversion device 132 therein is responsible for converting the optical signal into an electrical signal. The signal processing unit 14 is responsible for processing the collected signal and outputting a scattered light image and a fluorescence image, wherein the scattered light image comprises bright-field, dark-field and side scattering images, and the fluorescence image may comprise 1-50 fluorescent channel signals. The spatially coded reagents 2 can be composed of spatially arranged fluorescent microspheres of different colors. Depending on the colors and spatial positions of the fluorescent microspheres, the spatially coded reagents 2 can generate different coding sequences, thereby forming a coding sequence library 21. Each spatially coded reagent is assigned and specifically binds to a biological particle to be detected. Biological particles 3 labeled with spatially coded reagents 2 together constitute the analytes 15 in the imaging flow cytometer 1. The imaging flow cytometer 1 can observe and distinguish different spatially coded reagents 2, thereby determining the type of biological particle 3 specifically coupled to the spatially coded reagents. The coding sequence library 21 and a biological particle library 31 are constructed, and each biological particle in the biological particle library is assigned a unique coding sequence. For example, in the event that coded reagents 00000-99999 specifically bind to biological particles 00000-99999, respectively, if reagent 12345 is observed in the imaging flow cytometer 1, it is seen that biological particle 12345 is under detection. Specifically, the biological particles 3 to be detected include, but are not limited to, nucleic acids, proteins, bacteria, viruses, exosomes and single cells. The detection process can detect only a certain type of biological particles, or simultaneously detect multiple different types of biological particles. To improve the detection accuracy, an electric field 122 can be applied along the direction of the flow channels 121 in the flow chamber 12. This aligns the direction of the length of the spatially coded reagents 2 with the direction of the flow channel, facilitating identification of the spatially coded reagents 2 and improving the detection accuracy of the imaging flow cytometer 1.

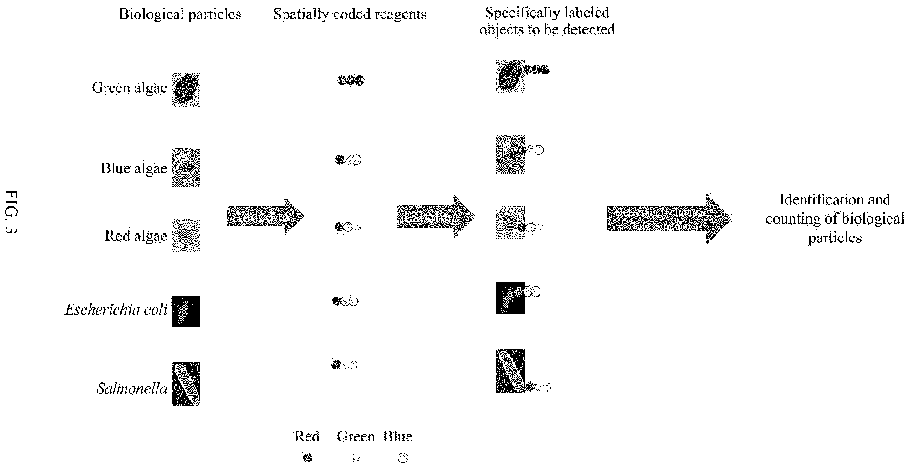

In the detection of water quality, it is necessary to detect green algae, blue algae, red algae, *Escherichia coli, and Salmonella.* As shown in FIG. 3, three-color coded reagents are designed: "Red-Red-Red," "Red-Green-Blue," "Red-Blue-Green," "Red-Blue-Blue," and "Red-Green-Green," corresponding to the five above analytes. By coupling with corresponding antibodies, the five spatially coded reagents can specifically bind to green algae, blue algae, red algae, *Escherichia coli,* and *Salmonella.* The imaging flow cytometer then rapidly identifies the five biological particles in the water body sample by distinguishing the colors of the spatially coded reagents. Data statistics are then generated according to the detection results to assess the water quality.

### EXAMPLE 3 SPATIALLY CODED REAGENTS FOR HIGH-THROUGHPUT SEQUENCING

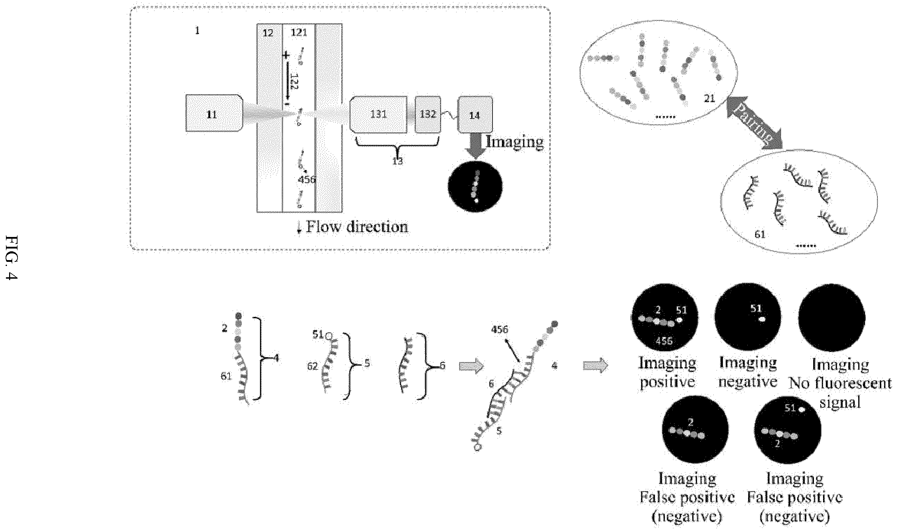

The sequencing method is based on imaging flow cytometry and spatially coded in Example 1. The specific steps are shown in FIG. 4.

Specifically, the detection method primarily relates to an imaging flow cytometer 1, modified spatially coded reagents 4, a reporter probe 5 for preventing false positives, and a single-stranded nucleic acid sequence 6 to be detected. The modified spatially coded reagents 4 consist of spatially coded reagents 2 and a segment of nucleic acid single strand 61. The reporter probe 5 consists of a fluorophore 51 and a segment of nucleic acid single strand 62, wherein the fluorophore 51 can be a different color from spatially coded reagents 2. One end of the nucleic acid single strand 61 that is not connected to the spatially coded reagents 2 contains a certain number of base sequences complementary to one end of the target sequence 6, and one end of the reporter probe that is not connected to the fluorophore 51 contains a certain number of base sequences complementary to the other end of the target sequence 6. In the detection, both the modified spatially coded reagents 4 and the reporter probe 5 complementarity bind to the single-stranded nucleic acid sequence 6 to be detected, forming a positive analyte 456. If the imaging flow cytometer 1 simultaneously displays fluorescence from the spatially coded reagents 2 and the reporter probe 5, it is indicated that the target sequence 6 has been detected. If the imaging flow cytometer 1 detects no fluorescence signal, or only detects the spatially coded reagent 2 or the reporter probe 5, or the spatially coded reagents 2 and the reporter probe 5 whose spatial positions are not in conformity to the design, it is indicated that the target sequence 6 has not been detected. For some sequence 6 to be detected, it is required to design corresponding modified spatially coded reagents 4 and reporter probe 5, and select different spatially coded reagents 2. A coding sequence library 21 and a nucleic acid library 61 are constructed, and each nucleic acid fragment in the nucleic acid library is assigned a unique coding sequence. Given that the spatially coded reagents 2 have a rich capacity, they enable the sequencing of numerous different nucleic acid sequences. This method can realize the sequencing of 10²-10⁵ different nucleic acid sequences, at a detection throughput of 10²-10⁵ nucleic acid sequences per second.

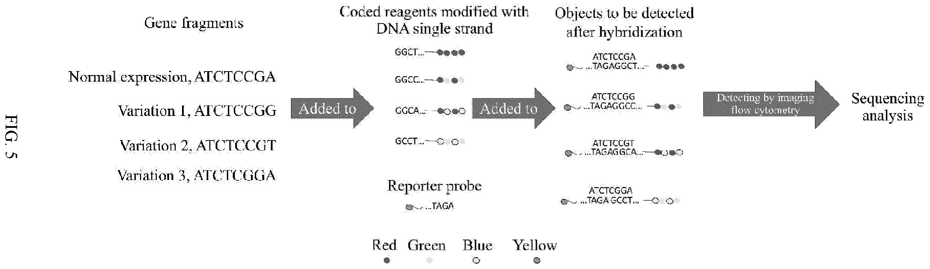

A normal human gene expression is ATCTCCGA, and common variants are expressed as ATCTCCGG, ATCTCCGT, and ATCTCGGA. As such, four spatially coded reagents for fluorescent microspheres are designed as "Red Red Red Red," "Red Green Red Green," "Red Blue Red Blue," and "Blue Green Blue Green," as shown in FIG. 5. The four spatially coded reagents are attached to one end of four segments of synthetically prepared DNA single strands. The base sequences at the other ends of these four synthetic DNA single strands are GGCT, GGCC, GGCA, and GCCT, respectively, corresponding to the right ends of the normal and variant human gene sequences. The reporter probe is designed to consist of a yellow fluorophore and a segment of nucleic acid single strands. The end of the nucleic acid single strand not attached to the fluorophore has the sequence TAGA. The prepared gene fragments are hybridized with the four spatially coded reagents modified with DNA single strands and the reporter probe and then detected by imaging flow cytometry. If the detected fluorescence image is "Red, Red, Red, Red + Yellow," it is indicated that the normal gene ATCTCCGA has been detected. If "Red, Green, Red, Green + Yellow," "Red, Blue, Red, Blue + Yellow," or "Blue, Green, Blue, Green + Yellow" is detected, it is indicated that the variant sequence ATCTCCGG, ATCTCCGT, or ATCTCGGA has been detected. If only "Red Red Red Red," "Red Green Red Green," "Red Blue Red Blue," and "Blue Green Blue Green" are detected, it is indicated false positives and the outcomes should not be taken into account. This method enables rapid diagnosis of genetic diseases. In practical applications, the types of fluorescent spatially coded reagents can be expanded, enabling the parallel detection of numerous different gene sequences.

### EXAMPLE 4. SPATIALLY CODED REAGENTS FOR HIGH-THROUGHPUT PROTEIN DETECTION

The method for detecting proteins is based on imaging flow cytometry and spatially coded reagents in Example 1. Proteins can be free protein molecules or proteins on the cell surface.

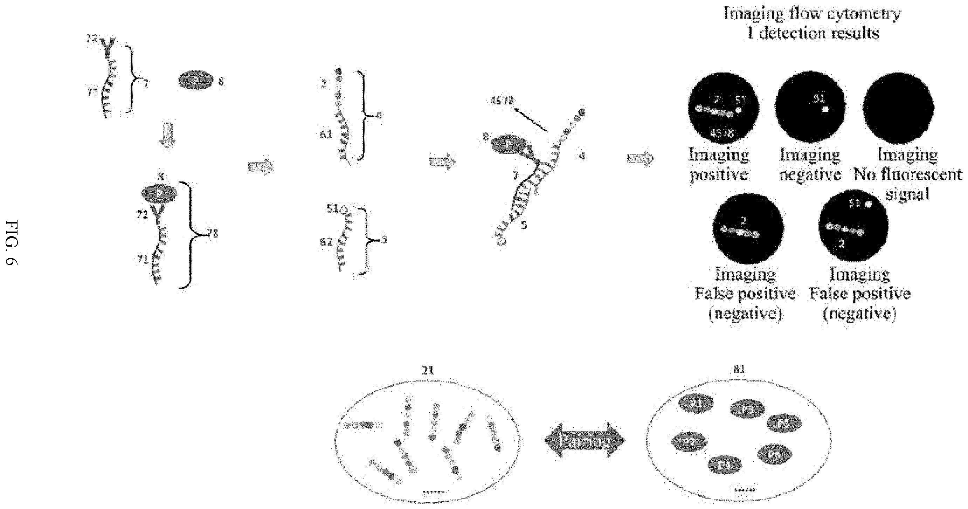

Specifically, for free protein molecules, the detection method is shown in FIG. 6. This detection method primarily comprises an imaging flow cytometer, modified spatially coded reagents 4, a reporter probe 5 for preventing false positives, a capture probe 7, and proteins 8 to be detected. The modified spatially coded reagents 4 consist of spatially coded reagents 2 and a segment of nucleic acid single strand 61. The reporter probe 5 consists of a fluorophore 51 and a segment of nucleic acid single strand 62, wherein the fluorophore 51 can be a different color from spatially coded reagents 2. The capture probe 7 comprises a segment of nucleic acid single strand 71 and an antibody 72 for capturing proteins. One end of the nucleic acid single strand 61 that is not connected to the spatially coded reagents 2 contains a certain number of base sequences complementary to one end of the probe nucleic acid single strand 71, and one end of the reporter probe that is not connected to the fluorophore 51 contains a certain number of base sequences complementary to the other end of the probe nucleic acid single strand 71. In the detection, the capture probe 7 captures the target proteins 8 via the antibody 72 to form a composition 78 to be detected. Both the modified spatially coded reagents 4 and the reporter probe 5 complementarily bind to the probe nucleic acid single strand 71 to be detected, forming a positive analyte 4578. If the imaging flow cytometer simultaneously displays fluorescent signals from the spatially coded reagents 2 and fluorescent signals 51 from the reporter probe 5, it is indicated that the target proteins 8 have been detected. If the imaging flow cytometer only detects the spatially coded reagent 2 or the reporter probe 5, or the spatially coded reagents 2 and the reporter probe 5 whose spatial positions are not in conformity with the design, it is indicated that the target proteins 8 have not been detected. For some target proteins 8, it is necessary to design corresponding modified spatially coded reagent 4, the reporter probe 5, and the capture probe 7, and select different spatially coded reagents 2. A coding sequence library 21 and a protein library 81 are constructed, and each protein in the protein library is assigned a unique coding sequence. Given that the spatially coded reagents 2 have a rich capacity, they enable the parallel detection of numerous different proteins. This method can realize the detection of 10²-10⁵ different proteins, at a detection throughput of 10²-10⁵ proteins per second. This method can be used in synthetic biology to detect cell-secreted protein products.

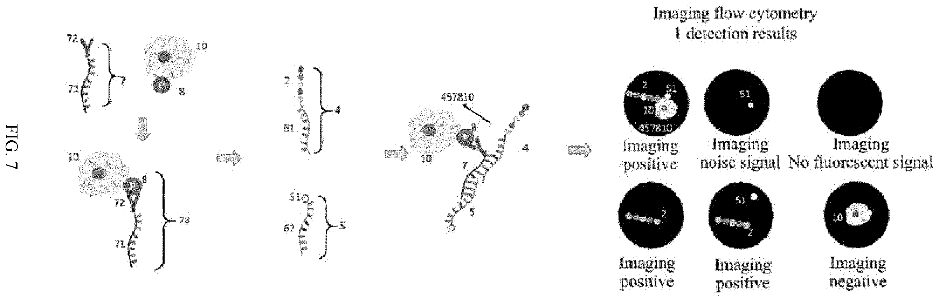

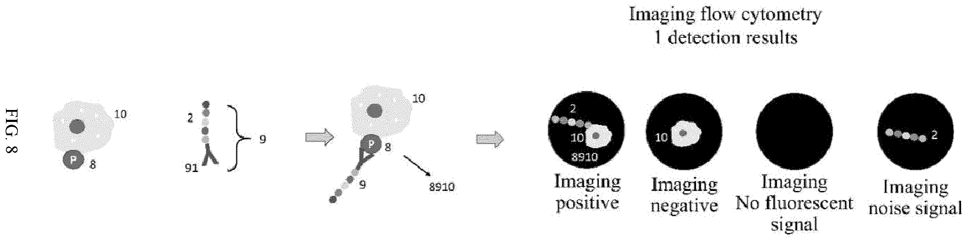

Surface proteins 8 of cells 10 can also be detected using the above method to form a positive analyte 457810, as shown in FIG.7. Alternatively, a simplified method can be used, as shown in Figure 8. This detection method primarily consists of an imaging flow cytometer, modified spatially coded reagents 9, and target proteins 8 on the surface of cells 10. The modified spatially coded reagents 9 consist of fluorescent spatially coded reagents 2 and an antibody 91 for binding to the target proteins 8. In the detection, the modified spatially coded reagents 9 bind to the target proteins 8 on the surface of the cells 10 via the antibody 91 therein, forming a positive analyte 8910. If the imaging flow cytometer simultaneously displays an image of cells 10 and the spatially coded reagents 2 bound to the cell surface, it is indicated that the target proteins 8 are present on the surface of the cells 10. Otherwise, it is indicated that the target proteins are absent. This method can be used to select cells expressing a specific membrane protein of interest.

### EXAMPLE 5. SPATIALLY CODED REAGENTS FOR SINGLE-CELL DETECTION

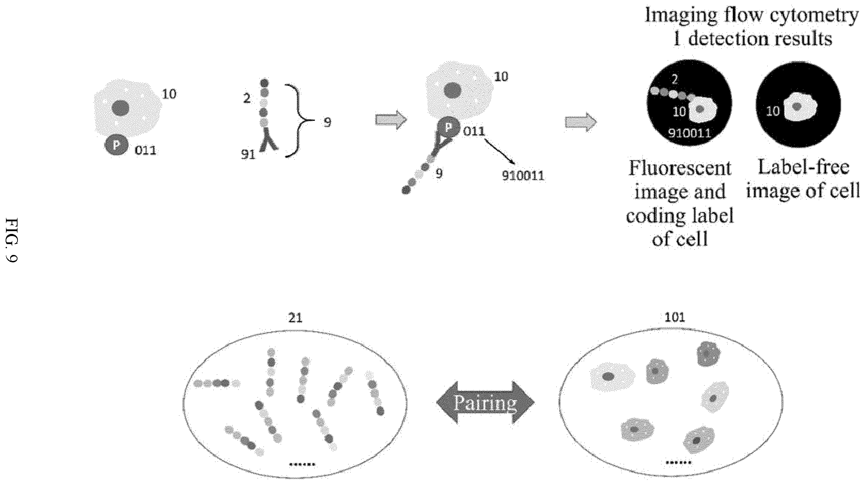

The method for detecting single cells is based on imaging flow cytometry and spatially coded reagents in Example 1. The specific steps are shown in FIG. 9.

Specifically, the detection method primarily relates to an imaging flow cytometer, modified spatially coded reagents 9, and anchoring proteins 011 on the surface of cells 10. The modified spatially coded reagents 9 consist of spatially coded reagents 2 and an antibody 91 for binding to the target anchoring proteins 011, forming a positive analyte 910011. In the detection, the modified spatially coded reagents 9 bind to the anchoring proteins 011 on the surface of the cells 10 via the antibody 91. The imaging flow cytometer can simultaneously display images of the cells 10 and the spatially coded reagents 2 bound to the cell surface. Cell images include label-free images and fluorescent labeling images. Label-free images include brightfield, darkfield, and scattered light images, and can directly measure morphological information of cells such as size, shape, and granularity. Fluorescent labeling images use different fluorescent probes to label the cell membrane, organelles, nucleus, or expressed proteins of cells, thereby generating image analysis information for cells. Imaging flow cytometry analyzes single cells based on the image information of cells. Given that the spatially coded reagents 2 have a rich capacity, they enable the labeling of numerous different cells. A coding sequence library 21 and a cell library 101 are constructed, and each cell/batch in the cell library is assigned a unique coding sequence. For example, if coding sequence 2 contains fluoresceins of 10 different colors and has 4 or 5 spatial positions, 10⁴ or 10⁵ different coding sequences can be formed, enabling labeling of 10⁴ or 10⁵ cells per batch. In synthetic biology and drug screening, it is often required to screen cells that have undergone different cultures or treatments on a large scale. This labeling method can label cells that have undergone different cultures or treatments, and accurately associate the detection information with each cell. This allows single cells that meet preset criteria to be accurately found after cell sorting for the subsequent incubation or research.

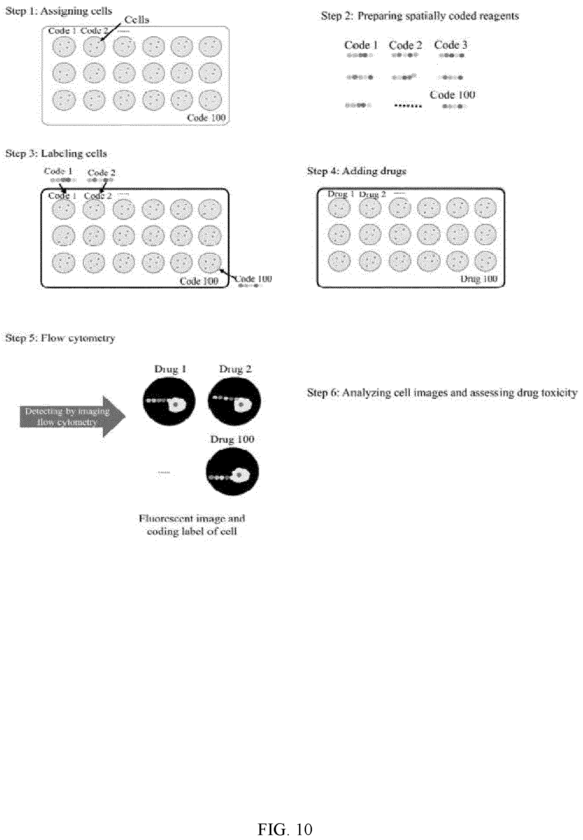

For example, it is often required to analyze drug toxicity during drug screening. As shown in FIG. 10, initially healthy human tissue cells are divided into 100 fractions, each containing about 100,000 cells. Each cell in each fraction is labeled with a unique spatially coding sequence. These 100 fractions are then exposed to 100 candidate drugs to study their cytotoxicity. Using imaging flow cytometry, the structures of single cells like the nucleus are observed to analyze cytotoxicity. By detecting the spatially coded reagents on each cell, it is possible to accurately track which candidate drug has been applied to the cell.

### EXAMPLE 6. SPATIALLY CODED REAGENTS FOR HIGH-THROUGHPUT PARALLEL DETECTION OF LIQUID BIOPSY SAMPLE

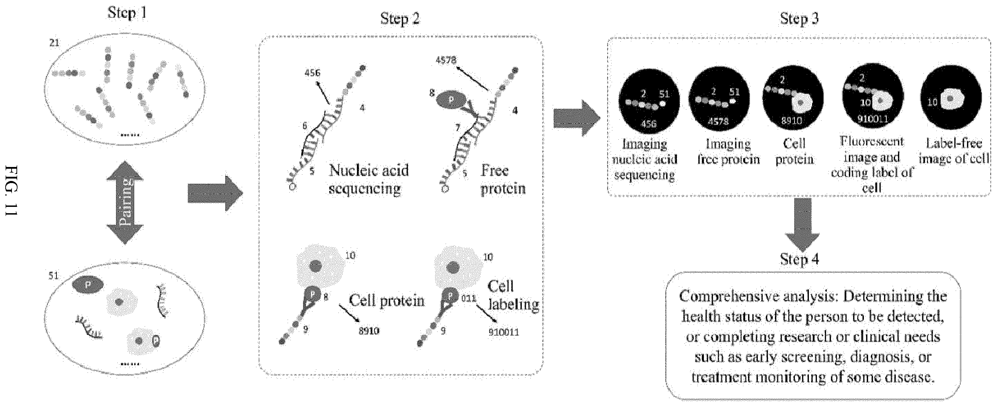

This liquid biopsy method is based on imaging flow cytometry and the method for high-throughput parallel detection of biological particles in Example 2. The specific steps are shown in FIG. 11.

Liquid biopsy requires parallel detection of different biomarkers. A comprehensive analysis of different markers can be used to determine the health status of the person to be detected, or to complete early screening, diagnosis, or treatment monitoring of some disease. The two most common categories of markers for detection in liquid biopsies include circulating tumor DNAs (ctDNAs) and circulating tumor cells (CTCs). Other markers further include free RNAs or DNAs (cfRNAs or cfDNAs), exosomes, and free proteins. That is, liquid biopsies involve nucleic acid sequencing, protein detection, and single-cell analysis. The first step is to construct a coding sequence library 21 and a biological library 51; uniformly code all target analytes to be detected in parallel in the biological library 51, and assign each target marker a unique code, thereby achieving accurate identification of each target marker. The second step is to design and prepare the respective detection reagent having spatially coded reagents. The specific method is as described above. The third step is to perform imaging flow cytometric detection. The specific detection method is as described above. The fourth step is to comprehensively analyze the detection results and draw conclusions.

In the description of this specification, reference to the terms "one/an example", "some examples", "exemplification", "specific exemplification", "some embodiments" or "some exemplifications" etc. means that the specific features, structures, materials or characteristics described in conjunction with the example or exemplification are included in at least one example or exemplification of the present invention. In this specification, the exemplary expressions of the above terms do not necessarily refer to the same example(s) or exemplification(s). Moreover, the specific features, structures, materials or characteristics described may be combined in any one or more examples or exemplifications in a suitable manner. In addition, those skilled in the art may combine and associate different examples or exemplifications and features in different examples or exemplifications described in this specification without mutual contradiction.

Although the examples of the present invention have been illustrated and described above, it would be appreciated that the above examples are exemplary and cannot be construed as limitations to the present invention. Any changes, modifications, alternatives, and variations can be made by those skilled in the art to the above examples within the scope of the present invention.

## Claims

1. A method for high-throughput parallel detection of biological particles, comprising:
(1) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library;
(2) constructing a biological particle library using known biological particles, and assigning one unique spatially coded reagent in the coding sequence library to each biological particle in the biological particle library to establish one-to-one correspondences between the coding sequence library and the biological particle library;
(3) labeling biological particles to be detected with the spatially coded reagents to form objects to be detected; and
(4) placing the objects to be detected in a suspended state in flow channels of an imaging flow cytometer for detection to obtain information of the biological particles to be detected.

2. The method according to claim 1, wherein the biological particles in step (2) comprise at least one of nucleic acids, proteins, bacteria, viruses, exosomes and single cells;
optionally, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres;
optionally, in step (3), the color type and spatial position arrangement of the microspheres labeled with the spatially coded reagents are consistent with the color type and spatial position arrangement of the microspheres of the spatially coded reagents in the coding sequence library corresponding to the biological particles to be detected;
optionally, the liquid flows in the flow channels of the imaging flow cytometer in step (4) have a central flow velocity of 0.1-20 m/s;
optionally, in step (4), the information of the biological particles to be detected is acquired by obtaining the color type and spatial position of the microspheres of the spatially coded reagents associated with the biological particles to be detected, and using the correspondence between the coding sequence library and the biological particle library.

3. A method for high-throughput sequencing, comprising:
(1) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library, wherein one end of the spatially coded reagent is connected to a nucleic acid single strand a, and the end of the nucleic acid single strand a that is not connected to the spatially coded reagent comprises complementary bases paired with a portion of bases in a single-stranded nucleic acid to be detected;
(2) connecting a fluorophore with a nucleic acid single strand b to prepare a reporter probe, wherein one end of the nucleic acid single strand b that is not connected to the fluorophore comprises complementary bases paired with a portion of the bases in the single-stranded nucleic acid to be detected, and in the single-stranded nucleic acid to be detected, the portion complementary to the nucleic acid single strand a does not overlap with the portion complementary to the nucleic acid single strand b;
(3) constructing single-stranded nucleic acid fragments to be detected into a nucleic acid sequence library, and assigning one unique spatially coded reagent in the coding sequence library to each nucleic acid fragment in the nucleic acid sequence library;
(4) simultaneously combining the spatially coded reagents and the reporter probe with single-stranded nucleic acid sequences to be detected to form objects to be detected, wherein the spatially coded reagents are types of spatially coded reagents corresponding to the types of all nucleic acid sequences in the nucleic acid sequence library; and
(5) placing the objects to be detected in a suspended state in flow channels of an imaging flow cytometer for detection to obtain information of nucleic acid sequences using the correspondences between the coding sequence library and the nucleic acid sequence library when the color type and spatial position of the microspheres labeled with the spatially coded reagents and the fluorophore are detected simultaneously.

4. The method according to claim 3, wherein the liquid flows in the flow channels of the imaging flow cytometer in step (5) have a central flow velocity of 0.1-20 m/s;
optionally, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

5. A method for high-throughput detection of proteins, comprising:
Method 1: (1) connecting an antibody and a nucleic acid single strand c to prepare a capture probe, wherein the antibody specifically binds to a target protein;
(2) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein one end of the spatially coded reagent is connected to a nucleic acid single strand d, and the end of the nucleic acid single strand d that is not connected to the spatially coded reagent comprises complementary bases paired with a portion of the bases in the nucleic acid single strand c;
(3) connecting a fluorophore with a nucleic acid single strand e to prepare a reporter probe, wherein the end of the nucleic acid single strand e that is not connected to the fluorophore comprises complementary bases paired with a portion of the bases in the nucleic acid single strand c;
and in the nucleic acid single strand c, the portion complementary to the nucleic acid single strand d does not overlap with the portion complementary to the nucleic acid single strand e;
(4) constructing proteins to be detected into a protein library, and assigning one unique spatially coded reagent in the coding sequence library to each protein in the protein library;
(5) simultaneously combining the spatially coded reagents and the reporter probe with the nucleic acid single strand c in the capture probe to form objects to be detected; and
(6) placing the objects to be detected in a suspended state in flow channels of an imaging flow cytometer for detection to obtain information of proteins using the correspondences between the coding sequence library and the protein library when the color type and spatial position of the microspheres labeled with the spatially coded reagent and the fluorophore are detected simultaneously;
or Method 2: (1) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein the spatially coded reagents are connected to an antibody for specific binding to a target protein;
(2) constructing proteins to be detected into a protein library, and assigning one unique spatially coded reagent in the coding sequence library to each protein in the protein library;
(3) connecting the spatially coded reagents to the target protein on the cell surface through an antibody to form objects to be detected; and
(4) placing the objects to be detected in a suspended state in flow channels of an imaging flow cytometer for detection to obtain information of proteins using the correspondences between the coding sequence library and the protein library when the color type and spatial position of the microspheres labeled with the spatially coded reagents are detected.

6. The method according to claim 5, wherein the liquid flows in the flow channels of the imaging flow cytometer have a central flow velocity of 0.1-20 m/s;
optionally, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

7. A method for single-cell detection, comprising:
(1) specifically binding the surface of single cells to be detected to an anchor protein;
(2) selecting fluorescent labels having small fluorescence spectral overlap, labeling the surface of microspheres with the fluorescent labels, arranging and combining the labeled microspheres in space, followed by connecting them to form spatially coded reagents, and arranging the spatially coded reagents to form a coding sequence library; wherein the spatially coded reagents are connected to an antibody for specific binding to the anchor protein;
(3) constructing cells to be detected into a cell library, and assigning one unique spatially coded reagent in the coding sequence library to each cell in the cell library;
(4) connecting the spatially coded reagents to the anchor protein on the cell surface through an antibody to form objects to be detected; and
(5) placing the objects to be detected in a suspended state in flow channels of an imaging flow cytometer for detection to obtain information of single cells using the correspondence between the coding sequence library and the cell library when the color type and spatial position of the microspheres labeled with the spatially coded reagents are detected.

8. The method according to claim 7, wherein the liquid flows in the flow channels of the imaging flow cytometer have a central flow velocity of 0.1-20 m/s.
optionally, the microspheres comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres.

9. A method for high-throughput parallel detection of a liquid biopsy sample, comprising:
detecting biological particles contained in the liquid biopsy sample using the method of claim 1 or 2.

10. The method according to claim 9, wherein the liquid biopsy sample comprises at least one of cfDNA, a cell, free RNA, DNA, an exosome, and a free protein;
optionally, the method comprises:
sequencing nucleic acids contained in the liquid biopsy sample using the method for high-throughput sequencing of claim 3 or 4;
detecting proteins contained in the liquid biopsy sample using the method for high-throughput detection of proteins of claim 5 or 6;
detecting cells contained in the liquid biopsy sample using the method for single-cell detection of claim 7 or 8;
wherein the spatially coded reagents in the method for high-throughput sequencing, the method for high-throughput detection of proteins and the method for single-cell detection are different.

11. A method for preparing spatially coded reagents in the method of any one of claims 1-10, comprising:
(1) selecting fluorescent labels having small fluorescence spectral overlap, and labeling the surface of microspheres with the fluorescent labels, wherein each microsphere comprises only one fluorescent label on the surface;
(2) linking the surface of the labeled microspheres obtained in step (1) with DNA single-strands; and
(3) connecting the labeled microspheres by DNA complementary base pairing to obtain the spatially coded reagents.

12. The method according to claim 11, wherein the fluorescent labels in step (1) comprise at least one selected from fluorescein and quantum dots;
optionally, the fluorescent labels in step (1) are selected from 3 to 20 types;
optionally, the microspheres in step (1) comprise at least one selected from the group consisting of polystyrene microspheres, polyethylene microspheres, silicon microspheres, silica microspheres, magnetic microspheres, cross-linked polystyrene/polydivinylbenzene microspheres and polymethacrylate microspheres;
optionally, the spatially coded reagents are further modified, and the modification comprises connecting the spatially coded reagents to a nucleic acid single-stranded DNA or connecting the spatially coded reagents to an antibody.

13. Use of imaging flow cytometry and spatially coded reagents in the detection of biological particles, wherein the method for the detection of biological particles includes the method of claim 1 or 2.

14. The use according to claim 13, wherein the biological particles comprise at least one of nucleic acids, proteins, bacteria, viruses, exosomes and single cells.
